(19) Europäisches Patentamt
European Patent Office
Office européen des brevets

(11) Veröffentlichungsnummer: **0 004 958**
**B1**

(12) # EUROPÄISCHE PATENTSCHRIFT

(45) Veröffentlichungstag der Patentschrift: **26.05.82**

(21) Anmeldenummer: **79101157.0**

(22) Anmeldetag: **17.04.79**

(51) Int. Cl.³: **C 07 C 85/06,** C 07 C 93/04, C 07 C 87/62, B 01 J 23/86

(54) **Verfahren zur Herstellung von 2,6-Dialkyl-N-alkylanilinen.**

(30) Priorität: **26.04.78 US 899903**

(43) Veröffentlichungstag der Anmeldung: **31.10.79 Patentblatt 79/22**

(45) Bekanntmachung des Hinweises auf die Patenterteilung: **26.05.82 Patentblatt 82/21**

(84) Benannte Vertragsstaaten: **CH DE FR GB IT**

(56) Entgegenhaltungen: **CHEMICAL ABSTRACTS, vol. 88, 1978, 190362g, Seite 721. Columbus, Ohio, USA**

(73) Patentinhaber: **CIBA-GEIGY AG
Patentabteilung Postfach
CH-4002 Basel (CH)**

(72) Erfinder: **Radimerski, Paul, Dr.
5852 Hickory Ridge Boulevard
Baton Rouge Louisiana, 70816 (US)**
Erfinder: **Rusek, Milos
Holeeholzweg 59
CH 4102 Binningen (CH)**

**EP 0 004 958 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 2,6-Dialkyl-N-alkylanilinen

Die vorliegende Erfindung betrifft ein Verfahren zur Herstellung von 2,6-Dialkyl-N-alkylanilinen der Formel I

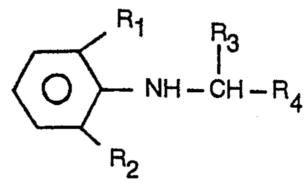

I

in welcher

$R_1$ und $R_2$ unabhängig voneinander je Methyl oder Aethyl

$R_3$ Wasserstoff, Methyl oder Aethyl und

$R_4$ eine Alkylgruppe mit 1 bis 11 Kohlenstoffatomen oder eine Alkoxymethylgruppe mit 1 bis 8 Kohlenstoffatomen in der Alkylgruppe bedeutet.

Die vorliegende Erfindung bezieht sich insbesondere auf ein Verfahren zur Herstellung von 2,6-Dialkyl-N-alkylanilinen der Formel I, in welcher $R_1$ und $R_2$ unabhängig voneinander je Methyl oder Aethyl, $R_3$ Wasserstoff oder Methyl und $R_4$ Alkoxymethyl mit 1 bis 3 Kohlenstoffatomen in der Alkylgruppe bedeuten.

2,6-Dialkyl-N-alkylaniline der Formel I sind wertvolle Zwischenprodukte zur Herstellung pestizider Wirkstoffe. So können 2,6-Dialkyl-N-alkylaniline der Formel I mit Halogenacetylhalogeniden, wie Chloracetylchlorid oder Bromacetylbromid in die entsprechenden Acetanilide mit pestizider Wirkung überführt werden. Solche Acetanilide, ihre Herstellung und Verwendung sind beispielsweise in der am 31. Januar 1973 eingereichten US-Patentanmeldung 328.202, in den US-Patentschriften 3.952.056 und 3.937.730, sowie in der deutschen Patentschrift 2.305.495 und der deutschen Auslegeschrift 2.328.340 beschrieben.

Es ist bekannt, N-Alkylaniline durch Umsetzung von Anilinen mit Alkylhalogeniden, -tosylaten oder -phosphaten herzustellen. Bei Anwendung dieser Methode werden jedoch ausser den gewünschten N-Monoalkylanilinen stets beträchtliche Mengen N,N-Dialkylaniline gebildet. Dieses Verfahren ist daher wegen seiner zu geringen Selektivität für eine technische Herstellung von Verbindungen der Formel I ungeeignet. Ausserdem wirft dieses Verfahren ökologische Probleme auf, da die Abwässer stets grosse Mengen von Halogenwasserstoff Toluolsulfonsäure oder Phosphorsäure bzw. von Salzen dieser Säuren enthalten.

Es ist ferner bekannt, Aniline durch Umsetzung mit Carbonylverbindungen in Gegenwart von Wasserstoff und wasserstoffübertragenden Katalysatoren herzustellen. Der wesentliche Nachteil dieses Verfahrens besteht darin, dass wegen zu geringer Selektivität erhebliche Mengen unerwünschter Nebenprodukte, wie N,N-Dialkylanilin und Kondensationsprodukte, entstehen, die die Ausbeute an gewünschtem N-Monoalkylanilin beeinträchtigen.

Es wurde ferner bereits vorgeschlagen, Aniline in Gegenwart von Katalysatoren mit Alkoholen zu N-Alkylanilinen umzusetzen (vgl. Kirk-Othmer, Encyclopedia of Chemical Technology, 2nd Edition, Vol. 2, 412—13). Dabei wurden als Katalysatoren neben Aluminiumoxyd, Aluminiumsilikat, einem Gemisch aus Phosphorsäure und Bentonit, Mineralsäuren, wie Chlorwasserstoff oder Schwefelsäure, auch wasserstoffübertragende Katalysatoren verwendet. Beispielsweise wird nach einem im US-Patent 2.580.284 beschriebenen Verfahren Anilin in Gegenwart eines kupferhaltigen Tonerdekatalysators und in Gegenwart von Wasserstoff mit Methanol in einer Ausbeute von 96% der Theorie zu N-Methylanilin umgesetzt. Ferner kann Aethanol in Gegenwart von Raney-Nickel in einer Ausbeute von 80 bis 83% der Theorie zu N-Aethylanilin umgesetzt werden. (vgl. J.Org.Chem. *21* 474—(1956) und J.Amer.Chem.-Soc. 77, 4052—54 (1955)). Bei der Umsetzung von Anilin und Methanol in Gegenwart von Kupferchromit und Wasserstoff wird in einheitlicher Reaktion N-Methylanilin in praktisch quantitativer Ausbeute gebildet (vgl. japanische Offenlegungsschrift 73 49,727; C.A. *79,* (1973), 136.769w und deutsche Offenlegungsschrift 2.061.709.

Wie die vorstehende Besprechung des Standes der Technik zeigt, führt die Umsetzung von Anilin mit Alkoholen in Gegenwart von Wasserstoff und Wasserstoffübertragenden Katalysatoren in ausgezeichneten Ausbeuten zu N-Alkylanilinen. Nach diesem Verfahren lassen sich jedoch 2,6-Dialkylaniline mit Alkoholen und insbesondere mit Alkoxyalkanolen nicht zu den entsprechenden 2,6-Dialkyl-N-alkylanilinen umsetzen.

Der vorliegenden Erfindung liegt daher die Aufgabe zugrunde, ein Verfahren bereitzustellen, nach dem 2,6-Dialkylaniline in guten Ausbeuten mit Alkoholen zu den entsprechenden 2,6-Dialkyl-N-alkylanilinen umgesetzt werden können.

Es wurde nun gefunden, dass man 2,6-Dialkyl-N-alkylaniline der Formel I in ausgezeichneter Ausbeute herstellen kann, wenn man ein 2,6-Dialkylanilin der Formel II

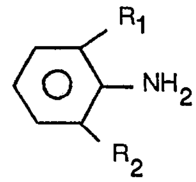

(II)

in welcher $R_1$ und $R_2$ die unter Formel I angegebene Bedeutung haben, in Gegenwart eines kupferhaltigen Katalysators de 0,05 bis 10 Gew.-% Palladium oder Platin enthält, bei 200 bis 350°C mit einem Alkohol der Formel III

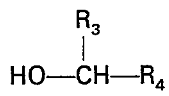

(III)

in welcher $R_3$ und $R_4$ die unter Formel I angegebene Bedeutung haben, umsetzt.

Als kupferhaltige Katalysatoren kommen allgemein handelsübliche Katalysatoren in Betracht, die Kupfer enthalten, z.B. Kupferoxid oder Gemische von Kupferoxid mit anderen Metalloxiden, wie Chromoxid oder Zinkoxid. Die Katalysatoren können ferner auf einem inerten Träger, wie Silicagel, Aluminiumoxid oder Silikaten, wie z.B. Magnesiumsilikat, niedergeschlagen sein und als weitere Zusätze geringe Mengen Erdalkalimetalloxide oder Alkalimetalloxide, wie Bariumoxid oder Natriumoxid, enthalten.

Besonders geeignet sind Kupferoxid-Chromoxid-Katalysatoren mit einem Molverhältnis von Kupferoxid, Chromoxid von 1:1 bis 15:1, die 0,05 bis 10 Gew.-%, vorzugsweise 0,2 bis 3 Gew.-% Palladium oder Platin enthalten. Katalysatoren dieser Art sind neu.

Die erfindungsgemäss zu verwendenden Katalysatoren können hergestellt werden, indem man einen handelsüblichen, kupferhaltigen Katalysator der vorgenannten Art mit einer Lösung eines Platin- oder Palladiumsalzes, wie z.B. Platin- oder Palladiumacetat, tränkt und anschliessend trocknet. Es ist jedoch auch möglich, die erfindungsgemäss zu verwendenden Katalysatoren in der Weise herzustellen, dass man einen handelsüblichen, kupferhaltigen Katalysator der vorgenannten Art einfach mit einer festen, Platin oder Palladium enthaltenden Substanz, wie z.B. Platinoxid, Platin auf Kohle, Palladiumacetat, Palladiumhydroxid auf Bariumsulfat und dergl., vermischt.

Ein besonders geeigneter, erfindungsgemäss zu verwendender Katalysator wird erhalten, wenn man aus einer etwa äquimolare Mengen eines Kupfer(II)salzes und eines Chrom(III)salzes enthaltenden, wässrigen Lösung zunächst durch Zugabe einer wässrigen Alkalicarbonatlösung, z.B. Kaliumcarbonatlösung, ein Gemisch von Kupferhydroxycarbonat und Chromoxid-hydrat abscheidet, dieses durch Waschen mit Wasser von Alkalimetallionen befreit, das durch Absaugen der Hauptmenge Wasser erhaltene Gemisch extrudiert, trocknet und mit einer Lösung eines Palladium- oder Platinsalzes tränkt und nochmals trocknet. Beispielsweise können die extrudierten Katalysatorteilchen mit einer Lösung von Palladiumacetat in Benzol getränkt werden. Die Herstellung des Katalysators kann jedoch auch in der Weise durchgeführt werden, dass man eine entsprechende Menge eines Platin- oder Palladiumsalzes zusammen mit dem Kupfer(II)salz und dem Chrom(III)salz in Wasser löst und durch Zugabe von Alkalicarbonatlösung das Platin bzw. Palladium gemeinsam mit Kupfer und Chrom als basisches Carbonat fällt. Man erhält so durch Extrudieren und Trocknen der ausgefällten Carbonate direkt den gebrauchsfertigen Katalysator. Als Kupfer(II)salze und Chrom(III)salze eignen sich beispielsweise die Chloride, Sulfate, Acetate und insbesondere die Nitrate.

Vor seiner erfindungsgemässen Verwendung wird der Katalysator mit Wasserstoff aktiviert. Diese Aktierung erfolgt in der Weise, dass man bei Temperaturen zwischen 120 und 350°C Wasserstoff oder ein Gemisch aus Wasserstoff und Stickstoff über den Katalysator leitet. Die anschliessende Umsetzung eines 2,6-Dialkylanilins der Formel II mit einem Alkohol der Formel III wird vorzugsweise bei einer Temperatur zwischen 220 und 300°C durchgeführt. Das erfindungsgemässe Verfahren wird bei Normaldruck oder unter einem Ueberdruck von bis zu 50 bar durchgeführt. Besonders günstige Resultate werden im Druckbereich von 2 bis 5 bar erhalten.

Das Molverhältnis von 2,6-Dialkylanilin der Formel II zu Alkanol der Formel III beträgt erfindungsgemäss 1:1 bis 1:3, vorzugsweise 1:1,5 bis 1:2,5.

Es ist vorteilhaft, die N-Alkylierung eines 2,6-Dialkylanilins der Formel II mi einem Alkanol der Formel III in Gegenwart von Wasserstoff durchzuführen. Die Menge an Wasserstoff hängt einerseits vom Siedepunkt der im Reaktionsgemisch vorliegenden Komponenten und andererseits vom Druck ab, bei dem das Verfahren durchgeführt wird. Bei Normaldruck wird die Umsetzung in vorteilhafter Weise in Gegenwart von 1 bis 3 Mol Wasserstoff pro Mol 2,6-Dialkylanilin der Formel II durchgeführt. Im bevorzugten Druckbereich von 2 bis 5 bar werden vorteilhaft 2 bis 10 Mol Wasserstoff pro Mol 2,6-Dialkylanilin der Formel II eingesetzt. Bei höheren Drucken können, insbesondere wenn hochsiedende Reaktionsprodukte auftreten, noch grössere Mengen an Wasserstoff eingesetzt werden.

Durch das erfindungsgemässe Verfahren wird es möglich, auch 2,6-Dialkylaniline, die bisher der Alkylierung mit Alkoholen nicht zugänglich waren, mit Alkoholen zu alkylieren. Dadurch können die

Zwischenprodukte der Formel I ausgehend von billigen, liecht zugänglichen Alkoholen mit hohem Umsatz und ausgezeichneter Selektivität hergestellt werden. Das Verfahren kann aufgrund der kurzen Reaktionszeiten leicht kontinuierlich durchgeführt werden und ist daher für eine technische Herstellung von Zwischenprodukten der Formel I gut geeignet. Das Verfahren bietet ferner gegenüber denjenigen Verfahren, bei denen die Alkylierung mit Alkylhalogeniden oder -tosylaten durchgeführt wird, in ökologischer Hinsicht wesentliche Vorteile, da die bei diesen Verfahren auftretende, hohe Salzbelastung des Abwassers wegfällt.

Das erfindungsgemässe Verfahren wird durch die nachfolgenden Beispiele näher erläutert.

Beispiel 1

Herstellung von 2,6-Dimethyl-N-(2′-methoxyäthyl)-anilin

Ein handelsüblicher, von Harshaw Chemie N.V., de Meern, Holland, hergestellter Katalysator (Cu-0203 T 1/8), der 78 Gew.-% Kupferoxid und 20 Gew.-% Chromoxid enthält, wird auf eine Partikelgrösse von 0,5 bis 0,8 mm zerkleinert, mit einer Palladiumacetat-Lösung getränkt und im Vakuum Trockenschrank bei 50 bis 120°C getrocknet. Der so hergestellte Katalysator enthält 0,2 Gew.-% Palladium.

In einem vertikal angeordneten, von einem Heizmantel umgebenen Quarzrohr von 30 cm Länge und 0,5 cm Durchmesser werden 2 ml dieses Katalysators in der in Beispiel 1 beschriebenen Weise bei 130°C aktiviert und anschliessend unter Wasserstoff auf 275°C erhitzt. Bei 275°C werden dann unter Normaldruck pro Stunde 1,21 g 2,6-Dimethylanilin, 1,52 g 2-Methoxyäthanol und 0,67 Nl Wasserstoff von oben nach unten durch den Reaktor geleitet. Das aus dem Reaktor austretende Gemisch wird kondensiert und destillativ aufgearbeitet.

Bei einer Versuchsdauer von 72 Stunden werden folgende Resultate erhalten:

| | |
|---|---|
| Gesamtumsatz bezogen auf 2,6-Dimethylanilin | 69,4% |
| Selektivität bezogen auf 2,6-Dimethylanilin | 80,8% |
| Umsatz zu 2,6-Dimethyl-N-(2′-methoxyäthyl)-anilin | 56,0% |
| Umsatz zu N-äthyliertem Produkt | 6,2% |
| Umsatz zu N,N-dialkyliertem Produkt | 0,8% |

Beispiel 2

Herstellung von 2,6-Dimethyl-N-(2′-methoxyäthyl)-anilin

In einem vertikal angeordneten Rohrreaktor aus Stahl von 110 cm Länge und 2 cm Durchmesser werden 100 ml eines Kupferoxid-chromoxid-Katalysators (Molverhältnis $CuO:Cr_2O_3 = 12:1$), der 1,5 Gew.-% Palladium enthält, im Stickstoff-Strom auf 130°C aufgeheizt und mit einem Stickstoff-Wasserstoff-Gemisch aktiviert, welches anfänglich 2 Vol.% Wasserstoff enthält. Während der Aktivierung wird der Anteil des Wasserstoffs im Stickstoff-Wasserstoff-Gemisch kontinuierlich erhöht, bis schliesslich reiner Wasserstoff über den Katalysator geleitet wird. Dann wird die Temperatur des Katalysators im Wasserstoff-Strom auf 275°C erhöht. Bei 250°C werden dann pro Stunde 60,6 g (0,5 Mol) 2,6-Dimethylanilin, 76 g (1 Mol) 2-Methoxyäthanol und 112 Nl (5 Mol) Wasserstoff mit einem Ueberdruck von 5 bar von oben nach unten durch den Reaktor geleitet. Das am unteren Ende des Reaktors austretende Reaktionsgemisch wird kondensiert und destillativ aufgearbeitet.

Nach einer Anlaufphase werden folgende Resultate erhalten:

| | |
|---|---|
| Gesamtumsatz bezogen auf 2,6-Dimethylanilin | 79,3% |
| Selektivität bezogen auf 2,6-Dimethylanilin | 92,4% |
| Gesamtumsatz bezogen auf 2-Methoxyäthanol | 41% |
| Selektivität bezogen auf 2-Methoxyäthanol | 90,6% |
| Umsatz zu 2,6-Dimethyl-N-(2′-methoxyäthyl)-anilin | 73,3% |
| Umsatz zu N-äthyliertem Produkt | 4,4% |
| Umsatz zu N,N-Dialkyliertem Produkt | 0,1% |

Beispiel 3

Herstellung von 2,6-Dimethyl-N-(2'-methoxyäthyl)-anilin

In einem adiabatischen Reaktor werden 100 ml eines Kupferoxid-chromoxid-Katalysators (Molverhältnis $CuO:Cr_2O_3 = 2:1$), der 1.5 Gew.-% Palladium enthält, in der in Beispiel 2 beschriebenen Weise aktiviert und im Wasserstoff-Strom um auf 275°C erhitzt. Dann werden bei 275°C und einem Druck von 4 bar (absolut) 60,6 g (0,5 Mol) 2,6-Dimethylanilin, 76 g (1,0 Mol) 2-Methoxyäthanol und 67,2 Nl (3 Mol) Wasserstoff durch das Katalysator-Bett geleitet. Das aus dem Reaktor austretende Reaktionsgemisch wird kondensiert und destillativ aufgearbeitet.

Nach Erreichen des stationären Zustandes werden folgende Resultate erhalten:

| | |
|---|---|
| Gesamtumsatz bezogen auf 2,6-Dimethyl-anilin | 81,5% |
| Selektivität bezogen auf 2,6-Dimethyl-anilin | 92,2% |
| Gesamtumsatz bezogen auf 2-Methoxyäthanol | 42,3% |
| Selektivität bezogen auf 2-Methoxyäthanol | 88,7% |
| Umsatz zu 2,6-Dimethyl-N-(2'-methoxyäthyl)-anilin | 75,1% |
| Umsatz zu N-äthyliertem Produkt | 5,2% |
| Umsatz zu N,N-dialkyliertem Produkt | 0,15% |

Beispiel 4

In einem vertikal angeordneten, mit einem Heizmantel versehenen Rohr aus Pyrex-Glas wird ein Mischkatalysator bestehend aus 74 Gew.-% Platin-Kohle (1 Gew.-% Pt) und 26 Gew.-% eines Kupferoxid-Chromoxid-Katalysators, der 78 Gew.-% Kupferoxid und 20 Gew.-% Chromoxid enthält (Harschaw Cu-0203 T), bei 200°C zunächst mit einem Stickstoff-Wasserstoff-Gemisch und dann mit reinem Wasserstoff aktiviert. Ueber den im Wasserstoff-Strom auf 220°C erhitzten Katalysator werden dann pro g Katalysator und Stunde bei Normaldruck 0,35 g eines Gemisches aus 53 Gew.-% 2-Aethyl-6-methyl-anilin und 47 Gew.-% Isopropanol (Molverhältnis 2-Aethyl-6-methyl-anilin zu Isopropanol = 1:2) und gleichzeitig 67,2 Nl (3 Mol) Wasserstoff pro Mol 2-Aethyl-6-methylanilin geleitet.

Die gaschromatographische Untersuchung des aus dem Reaktor austretenden Reaktionsgemisches ergibt einen Gesamtumsatz an 2-Aethyl-6-methyl-anilin von 30% und eine Ausbeute an 2-Aethyl-6-methyl-N-isopropylanilin von 95% der Theorie bezogen auf umgesetztes 2-Aethyl-6-methylanilin.

Beispiel 5

Herstellung von 2,6-Dimethyl-N-n-octyl-anilin

a) Herstellung des Katalysators

Einer gerührten Lösung von 1,6 kg Kupfer(II)nitrattrihydrat und 2,6 kg Chrom(III)nitrat-nonahydrat in 26 l deionisiertem Wasser von Zimmertemperatur wird eine Lösung von 2,6 kg Kaliumcarbonat in 20 l deionisiertem Wasser zugesetzt, wobei eine Suspension mit einem pH von etwa 7,5 entsteht. Nach beendigter Zugabe der Kaliumcarbonat-Lösung wird die entstandene Suspension 30 Minuten gerührt. Anschliessend lässt man absitzen und zieht die Mutterlauge ab. Der Rückstand wird in 30 l deionisiertem Wasser suspendiert. Danach lässt man wieder absitzen und zieht das Waschwasser ab. Diese Waschoperation wird wiederholt bis mit dem "Merckoquant"-Nitrat-Test (Katalog Nr. 10020) kein Nitrat nehr nachgewiesen werden kann. Der Wasserverbrauch liegt etwa bei 500 l und die Nitratkonzentration liegt nach dem Waschen unter 10 ppm.

Der Niederschlag wird dann auf einen Büchner-Trichter trocken abgesaugt, wobei ein Filterkuchen mit einem Wassergehalt von etwa 90 Gew.-% erhalten wird. Der in Form einer Paste erhaltene Filterkuchen wird dann extrudiert und das Extrudat 16 Stunden in einem Vakuum-Trockenschrank bei 160°C getrocknet. Nach dem Trocknen werden die extrudierten Stränge in kleine Stücke zerbrochen.

Anschliessend werden 200 g (1 l) des getrockneten Extrudats mit einer Lösung von 3,55 g Palladiumacetat in 600 ml Benzol versetzt. Die Mischung wird in einen Rotationsverdampfer überführt und das Benzol bei einer maximalen Temperatur von 50°C und einem Druck von 300 Torr abgedampft. Nach der Entfernung des Benzols wird der Katalysator in einem Vakuum-Trockenschrank bei einem Druck von 20 Torr und einer Temperatur von 50°C 2 Stunden getrocknet. Anschliessend wird die Temperatur im Laufe von 4 bis 6 Stunden auf 120°C erhöht und der Katalysator wird noch eine halbe Stunde bei 120°C getrocknet. Man erhält auf diese Weise einen Kupferchromid-Katalysator, der etwa 0,8 Gew.-% Palladium enthält.

b) Herstellung von 2,6-Dimethyl-N-n-octyl-anilin

In einem vertikal angeordneten, mit einem Heizmantel versehenen Rohr aus Pyrex-Glas wird der nach der vorstehenden Methode a) hergestellte Katalysator zunächst im Stickstoff-Strom auf 250°C erhitzt und anschliessend während 16 Stunden mit einem Stickstoff-Wasserstoff-Gemisch mit einem Wasserstoffgehalt von 3,3 Vol.% während 16 Stunden aktiviert. Anschliessend werden am Kopf des Reaktors bei 270 bis 275°C und Normaldruck pro g Katalysator und Stunde 1,8 g eines aus 31,7 Gew.-% 2,6-Dimethylanilin und 68,3 Gew.-% n-Octanol (Molverhältnis 2,6-Dimethylanilin zu n-Octanol = 1:2) und 89,6 Nl (4 Mol) Wasserstoff pro Mol 2,6-Dimethylanilin eindosiert.

Nach 40 stündigem Betrieb des Reaktors ergibt sich aufgrund von fortlaufend durchgeführten gaschromatographischen Analysen des aus dem Reaktor austretenden Produktes ein Gesamtumsatz von 2,6-Dimethylanilin von 63%. Die Ausbeute an 2,6-Dimethyl-N-n-octylanilin beträgt 90% der Theorie bezogen auf umgesetztes 2,6-Dimethylanilin.

Beispiel 6

Herstellung von 2,6-Dimethyl-N-äthyl-anilin

In einem vertikal angeordneten, mit einem Heizmantel versehenen Rohr aus Pyrex-Glas wird der nach der in Beispiel 5 beschriebenen Methode hergestellte Katalysator wie in Beispiel 6 beschrieben aktiviert. Anschliessend werden bei 275°C und Normaldruck pro g Katalysator und Stunde 1,8 g eines Gemisches aus 56,81 Gew.-% 2,6-Dimethylanilin und 43,19 Gew.-% Aethanol (Molverhältnis 2,6-Dimethylanilin zu Aethanol = 1:2) und 33,6 Nl (1,5 Mol) Wasserstoff pro Mol 2,6-Dimethylanilin von oben nach unten durch den Reaktor geleitet. Durch gaschromatographische Analyse des aus dem Reaktor austretenden Produktes ergibt sich eine Gesamtumsatz an 2,6-Dimethylanilin von 66% Die Ausbeute an 2,6-Dimethyl-N-äthyl-anilin beträgt 96% der Theorie bezogen auf umgesetztes 2,6-Dimethylanilin.

Beispiel 7

Herstellung von 2,6-Dimethyl-N-(2'-propoxyäthyl)-anilin

In einem vertikal angeordneten, mit einem Heizmantel versehenen Stahlrohr mit einem Durchmesser von 2,54 cm wird der nach Beispiel 5 a) hergestellte Katalysator mit einem Stickstoff-Wasserstoff-Gemisch mit einem Wasserstoffgehalt von 3,3 Vol.% während 16 Stunden bei 250°C aktiviert. Es werden insgesamt 60 ml Gasgemisch pro g Katalysator durchgesetzt. Anschliessend wird bei 270°C und einem Druck von 2 bar pro g Katalysator und Stunde 1,0 g eines Gemisches aus 2-Propoxyäthanol und 2,6-Diäthylanilin (Molverhältnis 2:1) zusammen mit 56 Nl (2,5 Mol) Wasserstoff pro Mol 2,6-Diäthylanilin über den Katalysator geleitet. Das aus dem Reaktor austretende Reaktionsgemisch, das aus 2-Propoxyäthanol, 2,6-Diäthylanilin, 2,6-Diäthyl-N-(2'-propoxyäthyl)anilin und Nebenprodukten besteht, wird fortlaufend gaschromatographisch analysiert. Aus den Analysendaten werden der Gesamtumsatz an 2,6-Diäthylanilin und die Selektivität berechnet. Die Ergebnisse sind in folgender Tabelle zusammengestellt:

| Versuchsdauer (h) | % Gesamtumsatz an 2,6-Diäthylanilin | Selektivität |
|---|---|---|
| 1–72 | 66 | 86,9 |
| 72–160 | 58,9 | 87,3 |
| 160–248 | 56,0 | 89,3 |
| 248–336 | 65,7 | 91,5 |

Beispiel 8

Alkylierung verschiedener Anilin-derivate mit 2-Methoxyäthanol mit einem Kupferoxid-Chromoxid-Katalysator ohne Zusatz von Platin oder Palladium

In einem vertikal angeordneten, mit einem Heizmantel versehenen Rohrreaktor aus Pyrex-Glas wird ein handelsüblicher Kupferoxid-Chromoxid-Katalysator, der 78 Gew.-% Kupferoxid und 20 Gew.-% Chromoxid enthält (Harschaw Cu-0203 T), unter Stickstoff auf 200°C erhitzt und dann mit einem Stickstoff-Wasserstoff-Gemisch aktiviert, das anfänglich 3,3 Vol.% Wasserstoff enthält. Während der Aktivierung wird der Wasserstoff-Anteil des Stickstoff-Wasserstoff-Gemisches kontinuierlich gesteigert, bis schliesslich reiner Wasserstoff über den Katalysator geleitet wird. Die Temperatur des Katalysators wird dann im Wasserstoff-Strom auf 250°C erhöht. Anschliessend werden am Kopf des Reaktors pro g Katalysator und Stunde 0,73 g (7,84 mMol) Anilin und 1,19 g (15,68 mMol) 2-Methoxyäthanol eindosiert. Das aus dem Reaktor austretende Reaktionsgemisch wird kondensiert und gaschromatographisch analysiert. Aufgrund der Analysendaten wird der Gesamtumsatz an Anilin und der Umsatz zu N-(2-methoxyäthyl)-anilin berechnet.

Anschliessend werden ohne Unterbrechung des Versuchs anstelle von Anilin unter gleichen Bedingungen pro g Katalysator und Stunde 0,73 g (6,81 mMol) o-Toluidin und 1,04 g (13,62 mMol) 2-Methoxyäthanol über den Katalysator geleitet. Das aus dem Reaktor austretende Reaktionsgemisch wird ebenfalls während der Versuchsdauer laufend gaschromatographisch analysiert. Aufgrund der Analysendaten werden wiederum der Gesamtumsatz an o-Toluidin und der Umsatz zu N-(2′-methoxyäthyl)-o-toluidin berechnet.

Dann werden anstelle von o-Toluidin unter gleichen Bedingungen pro g Katalysator und Stunde 0,73 g (6,02 mMol) 2,6-Dimethylanilin und 0,92 g (12,04 mMol) 2-Methoxyäthanol über den Katalysator geleitet und die Zusammensetzung des aus dem Reaktor austretenden Reaktionsgemisches gaschromatographisch analysiert. Aus den Analysendaten werden Gesamtumsatz an 2,6-Dimethylanilin und Umsatz zu 2,6-Dimethyl-(N-(2′-methoxyäthyl)-anilin berechnet.

Schliesslich werden zur Aktivitätskontrolle des Katalysators unter gleichen Bedingungen ohne Unterbrechung des Versuchs pro g Katalysator und Stunde nochmals 0,73 g (7,84 mMol) Anilin und 1,19 g (15,68 mMol) 2-Methoxyäthanol über den Katalysator geleitet und der Gesamtumsatz an Anilin sowie der Umsatz zu N-(2′-methoxyäthyl)-anilin bestimmt.

Die Resultate der einzelnen Versuch sind in der folgenden Tabelle zusammengefasst.

| Arom. Amin | Versuchsdauer [h] | % Gesamtumsatz an arom. Amin | % Umsatz zu N-(2′-methoxyäthyl) anilin |
|---|---|---|---|
| Anilin | 0–21 | 35 | 34 |
| o-Toluidin | 21–50 | 31 | 30 |
| 2,6-Dimethylanilin | 50–80 | 4 | 3,4 |
| Anilin | 80–100 | 33 | 32 |

Die Versuchsergebnisse zeigen, dass ohne den erfindungsgemässen Zusatz von Palladium oder Platin die Umsetzung von 2,6-Dimethylanilin und 2-Methoxyäthanol zu N-(2′-Methoxyäthyl)-2,6-dimethylanilin praktisch nicht durchführbar ist.

**Patentansprüche**

1. Verfahren zur Herstellung von 2,6-Dialkyl-N-alkylanilinen der Formel I

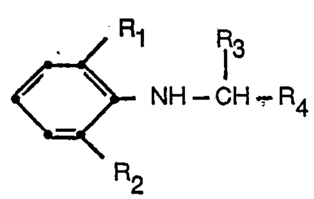

(I)

in welcher $R_1$ und $R_2$ je Methyl oder Aethyl, $R_3$ Wasserstoff, Methyl oder Aethyl und $R_4$ eine Alkylgruppe mit 1 bis 11 Kohlenstoffatomen oder eine Alkoxymethylgruppe mit 1 bis 8 Kohlenstoffatomen in der Alkylgruppe bedeutet, durch Umsetzung eines 2,6-Dialkylanilins der Formel II

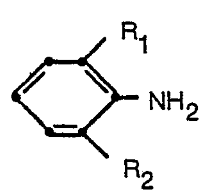

(II)

in welcher $R_1$ und $R_2$ die oben angegebene Bedeutung haben, mit einem Alkanol der Formel III

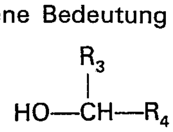

(III)

in welcher $R_3$ und $R_4$ die oben angegebene Bedeutung haben, in Gegenwart von Wasserstoff und einem wasserstoffübertragenden Katalysator, dadurch gekennzeichnet, dass man einen kupferhaltigen Katalysator verwendet, der 0,05—10 Gew.-% Palladium oder Platin enthält.

2. Verfahren nach Anspruch 1, dadurch gekennzeichnet, dass man die Umsetzung eines 2,6-Dialkylanilins der Formel II mit einem Alkohol der Formel III in Gegenwart von Wasserstoff durchführt.

3. Verfahren nach Anspruch 1, dadurch gekennzeichnet, dass man die Umsetzung eines 2,6-Dialkylanilins der Formel II mit einem Alkohol der Formel III bei einer Temperatur zwischen 220 und 300°C durchführt.

4. Verfahren nach Anspruch 1, dadurch gekennzeichnet, dass man die Umsetzung eines 2,6-Dialkylanilins der Formel II mit einem Alkohol der Formel III bei einem Druck von 2 bis 5 bar durchführt.

5. Verfahren nach Anspruch 1, dadurch gekennzeichnet, dass man die Umsetzung eines 2,6-Dialkylanilins der Formel II mit einem Alkohol der Formel III bei Normaldruck in Gegenwart von 1 bis 3 Mol Wasserstoff pro Mol 2,6-Dialkylanilin der Formel II durchführt.

6. Verfahren nach Anspruch 1, dadurch gekennzeichnet, dass man die Umsetzung eines 2,6-Dialkylanilins der Formel II mit einem Alkohol der Formel III bei einem Druck von 2 bis 5 bar in Gegenwart von 2 bis 10 Mol Wasserstoff pro Mol 2,6-Dialkylanilin der Formel II durchführt.

7. Verfahren nach Anspruch 1, dadurch gekennzeichnet, dass man die Umsetzung eines 2,6-Dialkylanilins der Formel II mit einem Alkohol der Formel III in Gegenwart eines Kupferoxid-Chromoxid-Katalysators mit einem Molverhältnis von Kupferoxid:Chromoxid von 1:1 bis 15:1 der 0,05 bis 10 Gew.-%, vorzugsweise 0,2 bis 3 Gew.-% Palladium oder Platin enthält, durchführt.

**Claims**

1. A process for producing a 2,6-dialkyl-N-aniline of the formula I

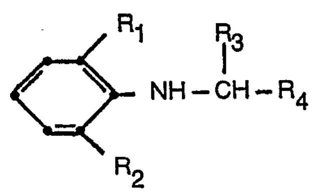

(I),

in which $R_1$ and $R_2$ are each methyl or ethyl, $R_3$ is hydrogen, methyl or ethyl, and $R_4$ is an alkyl group having 1 to 11 carbon atoms, or an alkoxymethyl group having 1 to 8 carbon atoms in the alkyl group, which process comprises reacting a 2,6-dialkylaniline of the formula II

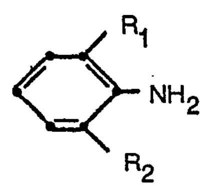

(II)

wherein $R_1$ and $R_2$ have the meanings defined above, with an alkanol of the formula III

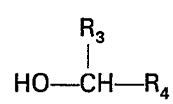

(III)

wherein $R_3$ and $R_4$ have the meanings defined above, in the presence of hydrogen and a hydrogen-transfering catalyst, characterised in that there is used a copper-containing catalyst which contains 0.05—10 per cent by weight of palladium or platinum.

2. A process according to Claim 1, wherein the reaction of a 2,6-dialkylaniline of the formula II with an alcohol of the formula III is performed in the presence of hydrogen.

3. A process according to Claim 1, wherein the reaction of a 2,6-dialkylaniline of the formula II with an alcohol of the formula III is performed at a temperature of between 220 and 300°C.

4. A process according to Claim 1, wherein the reaction of a 2,6-dialkylaniline of the formula II with an alcohol of the formula III is performed under a pressure of 2 to 5 bars.

5. A process according to Claim 1, wherein the reaction of a 2,6-dialkylaniline of the formula II with an alcohol of the formula III is performed under normal pressure in the presence of 1 to 3 mols of hydrogen per mol of 2,6-dialkylaniline of the formula II.

6. A process according to Claim 1, wherein the reaction of a 2,6-dialkylaniline of the formula II with an alcohol of the formula III is performed under a pressure of 2 to 5 bars in the presence of 2 to 10 mols of hydrogen per mol of 2,6-dialkylaniline of the formula II.

7. A process according to Claim 1, wherein the reaction of a 2,6-dialkylaniline of the formula II with an alcohol of the formula III is performed in the presence of a copper oxide/chromium oxide catalyst having a molar ratio of copper oxide to chromium oxide of 1:1 to 15:1 and containing 0.05 to 10 per cent by weight, preferably 0.2 to 3 per cent by weight, of palladium or platinum.

**Revendications**

1. Procédé de préparation des 2,6-dialcoyl-N-alcoylanilines de formule I

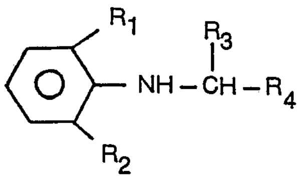

(I)

dans laquelle $R_1$ et $R_2$ représentent chacun un groupe méthyle ou éthyle, $R_3$ représente l'hydrogène, un groupe méthyle ou éthyle et $R_4$ représente un groupe alcoyle contenant 1 à 11 atomes de carbone ou un groupe alcoxyméthyle contenant 1 à 8 atomes de carbone dans le groupe alcoyle, par réaction d'une 2,6-dialcoylaniline de formule II

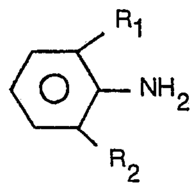

(II)

dans laquelle $R_1$ et $R_2$ ont les significations indiquées ci-dessus, avec un alcanol de formule III

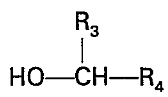

(III)

dans laquelle $R_3$ et $R_4$ ont les significations indiquées ci-dessus, en présence d'hydrogène et d'un catalyseur à transfert d'hydrogène, caractérisé en ce que l'on utilise un catalyseur contenant du cuivre et qui contient de 0,05 à 10% en poids de palladium ou de platine.

2. Procédé selon la revendication 1, caractérisé en ce que la réaction de la 2,6-dialcoylaniline de formule II avec un alcool de formule III est effectuée en présence d'hydrogène.

3. Procédé selon la revendication 1, caractérisé en ce que la réaction d'une 2,6-dialcoylaniline de formule II avec un alcool de formule III est effectuée à une température de 220 à 300°C.

4. Procédé selon la revendication 1, caractérisé en ce que la réaction d'une 2,6-dialcoylaniline de formule II avec un alcool de formule III est effectuée sous une pression de 2 à 5 bars.

5. Procédé selon la revendication 1, caractérisé en ce que la réaction d'une 2,6-dialcoylaniline de formule II avec un alcool de formule III est effectuée à pression normale en présence d'1 à 3 moles d'hydrogène par mole de la 2,6-dialcoylaniline de formule II.

6. Procédé selon la revendication 1, caractérisé en ce que la réaction d'une 2,6-dialcoylaniline de formule II avec un alcool de formule III est effectuée sous une pression de 2 à 5 bars en présence de 2 à 10 moles d'hydrogène par mole de la 2,6-dialcoylaniline de formule II.

7. Procédé selon la revendication 1, caractérisé en ce que la réaction d'une 2,6-dialcoylaniline de formule II avec un alcool de formule III est effectuée en présence d'un catalyseur oxyde de cuivre-oxyde de chrome à un rapport molaire oxyde de cuivre/oxyde de chrome de 1:1 à 15:1, qui contient de 0,05 à 10% en poids, de préférence de 0,2 à 3% en poids, de palladium ou de platine.